Europäisches Patentamt

(19) European Patent Office

Office européen des brevets

(11) **EP 0 611 163 B1**

(12) **EUROPEAN PATENT SPECIFICATION**

(45) Date of publication and mention
of the grant of the patent:
**20.10.1999 Bulletin 1999/42**

(51) Int. Cl.$^6$: **A01N 59/00**
// (A01N59/00, 25:34, 25:10)

(21) Application number: **94300999.3**

(22) Date of filing: **11.02.1994**

(54) **Polymeric biocidal composition and method for making same**

Polymere biozide Zusammensetzung und Verfahren zu ihrer Herstellung

Composition polymérique biocide et procédé pour sa préparation

(84) Designated Contracting States:
**AT BE CH DE DK ES FR GB GR IE IT LI LU MC NL
PT SE**

(30) Priority: **12.02.1993 US 16904**

(43) Date of publication of application:
**17.08.1994 Bulletin 1994/33**

(73) Proprietor:
**SOUTHWEST RESEARCH INSTITUTE
San Antonio Bexar County Texas (US)**

(72) Inventor:
**Wellinghoff, Stephen T.
San Antonio Bexar County Texas 78250 (US)**

(74) Representative:
**W.P. THOMPSON & CO.
Celcon House
289-293 High Holborn
London WC1V 7HU (GB)**

(56) References cited:
**WO-A-88/09176 US-A- 4 499 077
US-A- 4 585 482**

- **DATABASE WPI Week 8303, Derwent
Publications Ltd., London, GB; AN 83-06209K
[03] & JP-A-57 198 775 (NASA) 6 December 1982**
- **CHEMICAL ABSTRACTS, vol. 103, no. 20, 18
November 1985, Columbus, Ohio, US; abstract
no. 165940q, & JP-A-60 092 759 (JPN. KOKAI
TOKKYO KOHO) 24 May 1985**
- **DATABASE WPI Week 9229, Derwent
Publications Ltd., London, GB; AN 92-239876
[29] & JP-A-4 164 005 (TAKASUGI SEIYAKU) 9
June 1992**

EP 0 611 163 B1

Note: Within nine months from the publication of the mention of the grant of the European patent, any person may give
notice to the European Patent Office of opposition to the European patent granted. Notice of opposition shall be filed in
a written reasoned statement. It shall not be deemed to have been filed until the opposition fee has been paid. (Art.
99(1) European Patent Convention).

Printed by Xerox (UK) Business Services
2.16.7/3.6

## Description

### TECHNICAL FIELD OF THE INVENTION

The present invention relates generally to a composition for reducing bacterial and fungal growth and repelling insects, and more particularly to an ionomeric composition having a biocidal or insect repelling releasing agent and method for making same.

### BACKGROUND OF THE INVENTION

Chlorine dioxide ("$ClO_2$") is a well known bleach, disinfectant, fumigant and deodorizer [W. J. Masschelin and Rip G. Rice, "Chlorine Dioxide, Chemistry and Environmental Impact of Oxychlorine Compounds", Ann Arbor Science, Ann Arbor, Mich.] The biocidal uses of chlorine dioxide in packaging alone is a market of several hundred million dollars a year. Especially sought after are coatings which can release chlorine dioxide at surface concentrations of parts per million ppm over several months.

$ClO_2$ has been incorporated in polymer [JP 63,296,758, NOK Corp, 1988; CA 111 P120181s] and ceramic beads [JP 63,274,434, Enkler Busin.K.K., 1988; CA 111 P11975h], and in a calcium silicate wrapped in non-woven cloth [JP 57,168,977, Enkler Busin.K.K., 1982; CA 98 P77552] to be used as a deodorant. Food preservation is an application where a gel form is preferred JP-A-57022102, Diamaru Kogyo Kaishi, Ltd., 1982; CA 97 22409].

Chlorine dioxide generating gels have also been used as topical applications for disinfection [A.J. Kenyon, S.G. Hamilton and D.M. Douglas, Am.J.Vet.Res., 45(5), 1101 (1986)]. Typically the preparation involves mixing component A (gel with suspended $NaClO_2$) with component B (gel with lactic acid) immediately prior to use. $\alpha$-hydroxy carboxylic acids (citric, tartaric, malic, glycolic) are especially useful for generating $ClO_2$ by the reaction:

$$H^+ + NaClO_2 \rightarrow HClO_2 + Na^+ \quad 5HClO_2 \rightarrow 4ClO_2 + HCl + 2H_2O \qquad 1)$$

[Ger.Offen. 2,817,942, H.Allinger, 1979; WO-A-88/08823, J. Mason, 1988, CA 110 P98205h]. Alternatively, $NaClO_2$ and lactic acid have been separately encapsulated in polyvinyl alcohol and then mixed with water to generate $ClO_2$ [Can. 959,238, Chemical Generators, 1974]. Formulations involving sulfonic acids are also available [Europ. Pat. Appl. EP 287,074, Alcide Corp., 1987; CA 111 P45299f].

Food packaging with the incorporated disinfectant, chlorine dioxide, brings up an important public health question pertinent to this practice: Does chronic ingestion of residual levels of disinfectants result in a significant genetic or carcinogenetic hazard to the human population? It is apparent from published pharmacokinetic studies aided by incorporation of a chlorine radioisotope ($^{36}Cl$) that skin patch (Alcide gel) administration of chlorine dioxide and chlorite result in prolonged systemic residence of chlorine containing residues [J. Scatina, M. S. Aodel-Rahman, S.E. Gerges, Y. Khan and O. Gona, Fund.Appl.Tox., 4, 479 (1984)].

Meier et.al. published a report on the effect of subchronic and acute oral administration of chlorine, chlorine dioxide, sodium chlorite, and sodium chlorate on the induction of chromosomal aberrations and sperm-head abnormalities in mice [J.R. Meier, R.J. Bull, J.A. Stober and M.C. Cimino, Environ. Mutagenesis, 7, 201 (1985)]. Only the highly reactive hypochlorite resulted in a weak positive effect for mutagenic potential. The disinfectants failed to induce any chromosomal aberrations or increased numbers of micronuclei in the bone marrow of mice. Other hazards which have been associated with chlorine dioxide but were not incorporated in the published study include hemolytic anemia and hypothyroidism. One of the reasons for the relatively innocuous effect of $ClO_2$ is its inability to produce halomethanes, unlike hypochlorite and chlorine [R. Vilagines et al., Proc. AWWA Disinfect. Semin. 1977, paper 3, 24pp; CA 93 173513f].

Recently, the U.S. Patent No. 4,585,482 ('482) has used the gradual hydrolysis of alternating poly (vinyl methyl ether-maleic anhydride) or poly (lactic-glycolic acid) to generate acid which can release chlorine dioxide from sodium chlorite. In the process a solution process is used to encapsulate a polyalcohol humectant and water with the polyanhydride or polyacid in a nylon coating. After sodium chlorite is permitted to diffuse into the capsule through the nylon wall, an impermeable polystyrene layer is coacervated around the nylon capsule. The capsules can be coated onto surfaces to release chlorine dioxide and provide biocidal action for several days to months.

In the '482 patent it is also proposed to admix, for example, solid poly(lactic acid) and solid sodium chlorite to produce a composition which may be used as a biocide in air conditioning systems. Such a solid admixture is said to be stable until added to water when it reacts to form the biocidal agent.

Despite the many advantages of the '482 patent, there are some limitations. First, a large number of processing steps involving numerous chemical reactions and physical processes with disposal problems are required. Second, the chemistry is limited to batch processes. Third, a clear film cannot be produced. Finally, chlorine dioxide release starts immediately.

## SUMMARY OF THE INVENTION

The object of the present invention is to improve over the prior art by a method which is continuous, safer and has minimal number of preparation steps.

The more specific objects of the invention are: to provide a low cost, oligomer or polymer that can be coated onto surfaces as a film prior to conversion to a chlorite bearing substance. Also, the present invention provides a non-toxic oligomer or polymer that can be safely converted into a chlorite bearing film by exposure to chlorine dioxide, generated on site in gaseous form or in a non-aqueous solvent, eliminating the problem of storage of unstable chlorites. In addition, the invention provides a chlorite loaded film which will release chlorine dioxide over an extended period of time by exposure to moisture and both disinfect and repel insects from the surface onto which it is coated.

The present invention provides a long acting biocidal and insect repelling composition which employs a chlorine dioxide liberating polymer film which is converted to its stored chlorite form by exposure to chlorine dioxide in the presence of an acid precursor. In the presence of water the local pH in the polymer film is reduced, releasing the chlorine dioxide.

The present invention disclosed herein comprises a method for making a composition which liberates a biocidal and/or insect repellant agent which comprises the steps of synthesizing hydrogen bonded amines as a neat material and dissolving the amines into a hydrogen bonded matrix. Next, the amine is mixed with an incompatible phase containing a hydrolyzable phosphate ester or anhydride. By exposing the mixture to a gaseous chlorine dioxide, an ionomeric composition is created having an iminium chlorite salt-bound to the ionomeric composition such that said ionomeric polymer releases chlorine dioxide when exposed to water for acting as a biocidal agent.

Hence according to the present invention there is provided a composition for disinfection, reducing bacterial and fungal growth, and repelling insects comprising:

a first phase containing an iminium chlorite and a hydrogen-bonded matrix; and
a second phase containing a component that is hydrolyzable by atmospheric moisture to an acid, the first and second phases being in intimate contact and incompatible with one another, the first phase being capable of releasing chlorine dioxide upon hydrolysis of the hydrolyzable component on exposure to water.

The invention further provides a method of disinfecting, reducing bacterial and fungal growth, repelling insects from and/or deodorizing a substrate comprising:

exposing a substrate to a composition that releases chlorine dioxide in the presence of moisture; and
exposing the substrate to moisture to release chlorine dioxide from the composition into the atmosphere surrounding the substrate, the composition being comprised of a first phase containing iminium chlorite and a hydrogen-bonded matrix, and a second phase containing a component that is hydrolyzable by atmospheric moisture to an acid, the first and second phases being in intimate contact and incompatible with one another.

In accordance with yet a further aspect of the present invention there is provided a process for preparing a composition comprising:

providing a first phase containing an amine and a hydrogen-bonded matrix and a second phase containing a component that is hydrolyzable by atmospheric moisture to an acid, the first and second phases being in intimate contact and incompatible with one another; and
exposing the phases to chlorine dioxide that reacts with the amine to form iminium chlorite within the first phase, the first phase being capable of releasing chlorine dioxide upon hydrolysis of the hydrolyzable component.

As part of the invention, a composition is disclosed which liberates a biocidal and/or insect repelling agent which comprises a hydrogen bonded amine blended therein. The amine is grafted into hydrogen bonded matrices to create a mixture. An incompatible phase is mixed with the amine mixture. A gaseous chlorine dioxide exposed to said mixture creates an ionomeric composition such that the ionomeric composition releases chlorine dioxide when exposed to water for acting as a repelling agent or biocidal.

## BRIEF DESCRIPTION OF THE DRAWINGS

The present invention can be more readily understood by referring to the following figures in conjunction with the detailed description:

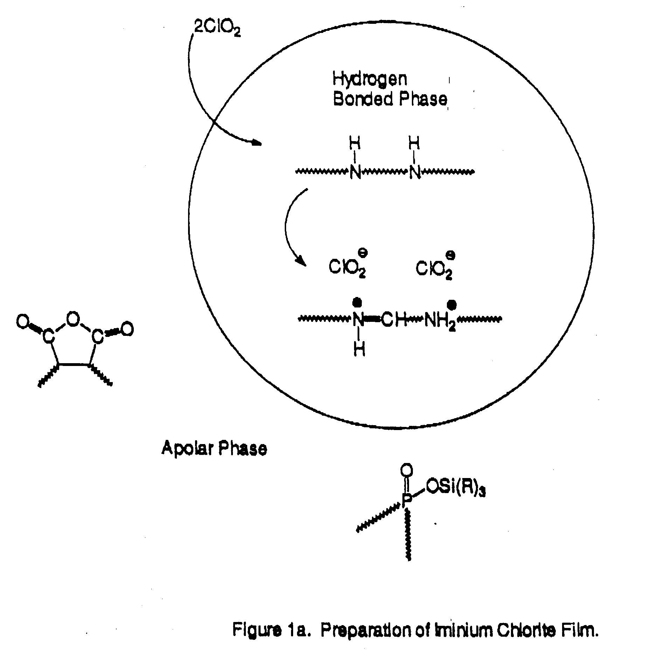

FIG. 1a illustrates the preparation of an iminium chlorite film; and

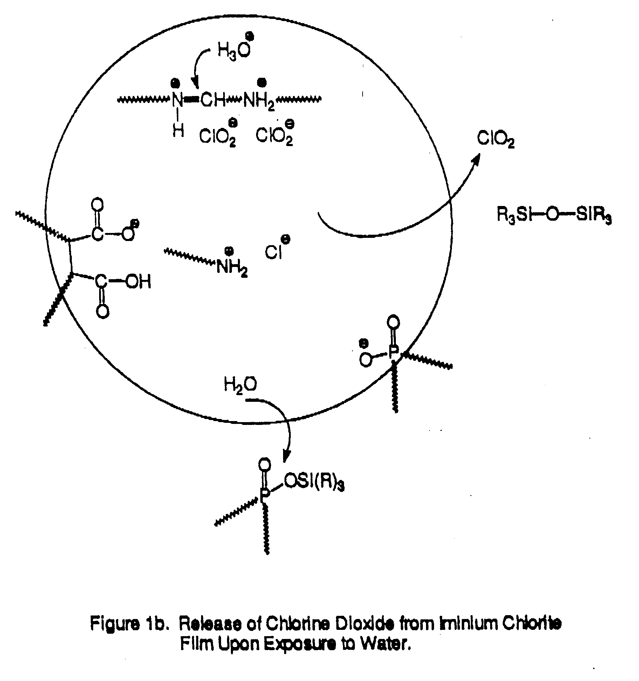

FIG. 1b illustrates the release of chlorine dioxide from iminium chlorite film upon exposure to water.

## DETAILED DESCRIPTION

Referring to FIGS. 1a and 1b, chlorine dioxide is a stable free radical that can undergo a redox reaction with organic amines to produce chlorite ion and an aminium radical cation. The radical cation quickly converts to an iminium cation by loss of a proton from the adjacent carbon atom and oxidation by another chlorine dioxide molecule [D.H. Rosenbatt et al., JACS, $\underline{89}$(5), 1158, 1163(1987); C.K. Chen, A.G. Hortmann and M.R. Marzabadi, JACS $\underline{110}$, 4829 (1988)]. The ionic species so created would phase separate into ionic microdomains which would provide humectant properties so that atmospheric moisture could be concentrated.

In the presence of water, at a sufficiently low pH, the iminium ion will cleave to an amine and an aldehyde. Each aldehyde has the potential to convert three chlorite anions to two chlorine dioxide molecules and a chloride anion with oxidation of the aldehyde to a carboxylic acid. The production rate of chlorine diode is of course enhanced by low pH. These protons can be supplied by the hydrolytic conversion of the anhydride or other acid generating moities to carboxylic acid or inorganic acids. All reacted groups can remain grafted to the polymer backbone after chlorine dioxide evolution and thus would not be released into the environment.

The above mechanism presents an easy and direct way to convert chlorine dioxide to chlorite in a polymer film and release it by stored moisture.

Chlorine dioxide can be continuously produced in 0.5% solution in water on a commercial scale. A water solution of chlorine dioxide will preferentially partition into a carbon tetrachloride phase with an equilibrium constant of 1.6 at 25°C [Int.Crit.Tab., $\underline{3}$, 419 (1928)]. Thus an efficient system for continuously reacting chlorine dioxide with the initial copolymer involves exchanging the chlorine dioxide from the water phase into an unreactive, hydrophobic solvent and expose the copolymer film to the organic solution saturated with chlorine dioxide. Since the diffusion constant and solubility for chlorine dioxide in the hydrophobic phase of the polymer phase will be much faster than water, the small amounts of moisture in the organic phase will be inconsequential.

The long acting chlorine dioxide releasing film of the present invention employs a "chlorine dioxide liberating compound" which is defined as the monomeric, oligomeric or polymeric iminium chlorite described above. The precursor amine to the iminium chlorite salt is dissolved in a hydrogen bonded oligomeric or polymeric matrix or suspended or emulsified in an apolar oligomeric or polymeric matrix. Alternatively, the matrix or the amine itself may be in a monomeric form which may be polymerized with or without grafting subsequent to coating onto the desired substrate.

In order for the amine functionality to react with chlorine dioxide to produce an iminium chlorite salt which may ultimately evolve chlorine dioxide, the amine nitrogen must be enclosed in a hydrogen bonded matrix The matrix must contain a sufficient molecular density of hydroxylic, -OH, or amide, -NHC(O)-, $H_2NC(O)$- groups, or amine, $H_xNR_{3-x}$, x=1,2. Possible matrices include formamide, acrylamide-isopropylacrylamide mixtures, water, glycerin, ethylene glycol or other oligoalkanols or oligoamides or primary or secondary oligoamines with sufficiently short apolar sections. Amine precursors to iminium chlorite salts can be of the form $H_xN(R)_{3-x}$, x=0,1,2, where R may be alkyl, cycloalkyl, pyridyl, benzyl, which can be dispersed as a separate phase in an apolar or polar non-hydrogen bonded matrix, which may be a monomeric, oligomeric or polymeric hydrocarbon.

Chlorine dioxide will react with the amines when amines of this type are dissolved to make one phase solutions in aliphatic or aromatic hydrocarbons or even in very polar solvents, such as dimethylformamide, dimethylsulfoxide, organic esters or acetonitrile, but in most cases no chlorite is formed and no chlorine dioxide can be released by acidification.

Neat primary and secondary amines with short non-hydrogen bonding groups will produce iminium chlorite which can release chlorine dioxide. However, neat tertiary amines with non-hydrogen bonding groups will react with chlorine dioxide, but will not produce a salt from which chlorine dioxide can be evolved.

An alternative strategy to assure reaction of the amine to form an iminium salt from which the chlorine dioxide can be released is to bond a hydrogen bonding group directly to the amine nitrogen. Thus, amines of the form $H_xN^1R_y^2R_{3-x-y}$ (x=0,1,2; R=$CH_2CH_2OH$,-$CH_2CH_2C(O)NH_2$) are especially preferred. Neat tertiary amines of this form will form chlorite salt from which chlorine dioxide can be released.

An effective chlorine dioxide introduction and reaction to chlorite occurs when the film is exposed above the $T_g$ (glass transition temperature) of the microphase in which the amine is dissolved.

The iminium chlorite salts can be induced to release chlorine dioxide by exposure to a hydrolyzed "acid generating compound" which includes carboxylic acids, esters, anhydrides, acyl halides, phosphoric acid and its trimethylsilyl esters, dialkyl phosphate and phosphate trimethylsilyl esters and sulfonic acids and hydrolyzable sulfonic esters and chlorides.

The acid anhydride can be in the form of a random copolymer of maleic anhydride and styrene or numerous other alkenes. Trimethylsilane esters of phosphates of the form $Me_3SiOP(O)(OR)_2$ (R = non hydrogen bonding groups, alkyl, aryl) are especially useful. Comonomers and R groups are selected so that the acid generating compound will preferentially dissolve in the non-hydrogen bonded phase and will not react with the amine containing hydrogen bonded phase and will not exude or extract into the environment. This may be accomplished by grafting the R group to the apolar polymer phase ,or increasing the molecular weight of the R groups.

Upon exposure to moisture the anhydride will form a dicarboxylic acid and the trimethylsilyl phosphate ester will form hexamethyldisiloxane and phosphoric acid or phosphoric acid ester. These acid species will migrate, due to interphase dispersal and compatibilization, to the hydrogen bonded phase to release chlorine dioxide from the iminium chlorite salt. The apolar phases containing the acid generating compound include low melting polyethylene waxes or atactic polypropylene. The hydrogen bonded phase containing the iminium chlorite salt also acts as a humectant ensuring that a critical amount of water is absorbed for the hydrolysis of the "acid generating compound".

The chlorite containing film can be manufactured as a hot melt adhesive or coating composition and hermetically sealed until use. In applications involving the simple coating of boxes, the polymer can be a low molecular weight form, heavily plasticized (e.g. by waxes or chlorinated waxes) so that the film could be applied as a tacky hot melt to the surface of the dry cardboard at the end of the cardboard manufacturing line.

The present invention is further illustrated by the following Examples.

EXAMPLE 1

This example illustrates the utility of primary and secondary hydrogen bonding amines as precursors to iminium chlorite in hydrogen bonded matrices. Hydrogen bonded matrices include the neat amine or amide type solvents. Hydroxylic solvents such as water, ethylene glycol and glycerine were also useful but had the disadvantage of reacting with the acid generating compound. Chlorine dioxide is released by exposure to hydrolyzed phosphate acid precursors.

Convenient non-aqueous media for the chlorine dioxide in larger scale practice are chlorinated paraffins of 70% chlorine content and $C_9$-$C_{15}$ fraction. These materials are non-toxic (suitable for food packaging), non-reactive with chlorine dioxide, and low viscosity, low vapor pressure liquids at room temperature. Their complete insolubility in water and high density of 1.30-1.36 g/cc would enable their easy separation from the chlorine dioxide saturated water phase. These compounds are also useful as fire retardant and plasticizer additives for hydrocarbon polymers. Their solubility parameter of 9.1 $(cal/cc)^{1/2}$ suggests that they would dissolve as much or more chlorine dioxide than carbon tetrachloride (8.6 $(cal/cc)^{1/2}$). The chlorinated paraffin is specially desirable because of its low fire danger. Its low vapor pressure would also permit it to be used in the vacuum loop of the chlorine dioxide generator in the place of water.

In smaller scale laboratory practice, volatile pentane was found to be more suitable. Thus $2.85X10^{-4}$ mole neat diethanolamine was exposed to $6X10^{-5}$ mole chlorine dioxide in pentane. The mixture remained two phase as the chlorine dioxide was completely absorbed in the amine to form a very viscous clear solution containing the iminium chlorite. Characteristic infrared absorbances for the chlorite anion were visible in the IR around 830 $cm^{-1}$. Absorbances for protonated amines were also visible. (Monoethanol and triethanol amines could both be converted to the iminium chlorite by a similar process).

The above process, utilizing the neat amines, was successful for virtually any primary or secondary amine which could be reacted as a phase separate from the chlorine dioxide solution.

This iminium chlorite solution was stable for at least one week at room temperature and exhibited a pH of 9. Up to one mole of chlorine dioxide per three moles of diethanol amine could be absorbed before the pH approached neutrality, precluding any more chlorine dioxide absorption. Long term stability of the solution was assured provided that the pH remained above 7-8, otherwise a slow decomposition of the iminium chlorite with a sustained release of chlorine dioxide would occur. Water appeared to enhance the release rate.

Bis (2-ethyl hexyl) phosphate (BCHP) is very soluble in pentane and other apolar media but is quite insoluble in highly hydrogen bonded solvents or ionomeric solutions such as formamide or the iminium chlorite. Thus, $3.0X10^{-4}$ mole BCHP solution in 1 ml pentane was added to the iminium chlorite with vortex mixing and the pentane pumped off under vacuum until a two phase dispersion resulted.

The interphase transfer of the BCHP was sufficient to induce a slow release of chlorine dioxide in the absence of water. However, the addition of 0.05 ml water accelerated the release rate. The initial release in the absence of water could be eliminated by dissolving the BCHP in mineral oil or other higher molecular weight hydrocarbon to minimize the interfacial acid concentration and decrease the diffusion rate through a viscosity increase.

Further improvements in the dry chemical stability of the two phase solution was achieved by dissolving the same amount of diethanol amine in formamide or a mixture of polymerizable acrylamide-isopropylacrylamide prior to chlorine dioxide exposure. Interestingly, the double bond in the acrylamides were stable to oxidation by chlorine dioxide. In these cases the phase segregation of the BCHP was complete enough that the BCHP could be added to the pentane solution of chlorine dioxide during the conversion to chlorite.

The acrylamide-isopropylacrylamide melt could be preoligomerized in the presence of the amine by means of heating to 60-70°C in the presence of ca. 0.01% azobisisobutyronitrile initiator. Provided that the film was sufficiently plasticized by the amine, formation of the iminium chlorite upon exposure of the film to the pentane-chlorine dioxide solution was still possible.

Other secondary, hydrogen bonded amines could be easily formed by the acetic acid catalyzed, addition of primary amines to Michael type substrates such as acrylamide and N,N methylene bisacrylamide. The secondary amines that could be converted to iminium chlorite in formamide or monomeric or oligomerized acrylamide-isopropylacrylamide mixtures include N,N methylene bis-[1-(N-cyclohexylamino) 2-carboxyamido] ethane and 1-(N-benzylamino) 2-carboxyamido ethane. Linear polymers formed by the coupling bifunctional primary amines such as 1,3 diamino propane and 1,2 diamino ethane to N,N methylene bisacrylamide could also be converted to iminium chlorite species in the same hydrogen bonded matrices.

Glassy polymer amine films which reacted only slowly with the chlorine dioxide could be induced to react to iminium chlorite in under 2 minutes provided that they were plasticized with at least 10 wt% low molecular weight amides.

EXAMPLE 2

This example illustrates the use of tertiary, hydrogen bonding amines as chlorite storage media in hydrogen bonded matrices in the presence of hydrolyzable trimethyl silyiphosphate esters.

Unlike primary and secondary amines which possess active hydrogens, tertiary amines will not react with anhydrides or trimethylsilyl phosphate esters. This permits the tertiary amines to be rather more intimately mixed with the "acid generating compound" providing for some processing advantages.

1-(N-dipropylamino), 2-carboxyamido ethane (DPACAE) was made by reacting 0.2 mole di(n-propyl)amine with 0.1 mole acrylamide in the presence of a small amount of acetic acid in 10 wt% solution methanol. The reaction was carried out for 3 hours at 70°C. After vacuum evaporation of the excess amine and crystallization in the presence of pentane, a white low melting solid was obtained ($T_m$ =60°C) which tended to lose amine and form acrylamide upon prolonged heating above the melting point.

1-(N-Dimethylamino), 2-carboxyamido ethane (DMACAE) was made by reacting 0.2 mole dimethylamine (as 40 wt% solution in water) with 0.1 mole acrylamide in 10 wt % solution in methanol. The reaction was carried out for 1 hour at room temperature. After vacuum evaporation of excess amine, methanol and water, the DMACAE was taken up in methylene chloride, dried with magnesium sulfate and isolated as a low melting ($T_m$ = 45°C) hydroscopic solid.

Both DPACAE and DMACAE crystallized only slowly and thus could be studied in the liquid state at room temperature. However, neither neat liquid formed iminium chlorite. 10-30% wt% solutions in formamide or acrylamide-isopropyl acrylamide readily formed iminium chlorite when exposed to chlorine dioxide.

Since the amide functionality is unreactive to anhydrides (acetic, propionic or succinic anhydride or anhydride containing polymers) or trimethylsilyl phosphate esters (tris-trimethylsilyl phosphate; O-trimethylsilyl bis-(2-ethyl hexyl) phosphate) at room temperature, DMACAE and DPACAE in formamide or acrylamides can be mixed with these acid generating compounds without reaction.

4-dimethylamino pyridine, tetramethylene ethylene diamine, and N,N dimethylamino cyclohexane also would form stable iminium chlorites in hydrogen bonding amide solvents. However, since both of these tertiary amines had considerable solubility in pentane, extraction of the amines into the apolar phase was avoided by layering the chlorine dioxide solution on the amide solution. None of these tertiary amines would form iminium chlorite when reacted in the pentane phase. However, they would react with the chlorine dioxide.

In practice it is still desirable to dissolve the silyl esters or anhydrides in an apolar hydrocarbon oligomer and disperse the apolar phase in the polar matrix containing either the amine or the imine chlorite. Even though the silyl esters and anhydrides do not react with tertiary amine they will react with the iminium chlorite reducing the ultimate yield of regenerated chlorine dioxide.

**Some important features of the invention are summarised in the following paragraphs.**

The method for making a composition which liberates a repelling or biocidal agent comprises the steps of:

synthesizing hydrogen bonded amines as a neat material;
dissolving said amines into hydrogen bonded matrices;
mixing the amine with an incompatible phase; and
exposing said mixture to gaseous chlorine dioxide to create an ionomeric composition having an iminium chlorite salt-bound to said ionomeric composition such that ionomeric polymer releases chlorine dioxide when exposed to water for acting as a biocidal agent.

The method may further comprise the step of creating said chlorine dioxide gas by dissolving chlorine dioxide in an organic solvent. The chlorine dioxide may be incorporated into n-pentane or other hydrocarbons or low melting chlorowaxes as a vehicle for reaction of the amine containing films to the iminium chlorite containing films.

The method may further comprise the step of selectively forming a film of ionomeric composition on a substrate.

The step of forming a film may comprise the steps of:

applying said ionomeric composition as a tacky hot melt to the substrate; and
controlling the operating temperature to avoid the decomposition temperature of any chlorite present.

The method may further comprise the step of lowering the pH of said ionomeric polymer to less than 8.0.

The composition which liberates a repelling or biocidal agent may comprise:

a hydrogen bonded amine grafted or mixed into hydrogen bonded matrices to create a mixture;
an incompatible phase mixed with the amine mixture; and
a gaseous chlorine dioxide exposed to said mixture to create an ionomeric composition such that said ionomeric composition releases chlorine dioxide when exposed to water for acting as a repelling or biocidal agent.

The hydrogen bonded amine may comprise a primary, secondary or tertiary hydrogen bonded amine and may be a monomeric amine, a polymeric amine or a mixture thereof.

The matrices may comprise aliphatic amides, aromatic amides, aromatic alcohols, aliphatic alcohols and mixtures thereof and may be selected from formamide, acrylamide-isopropylacrylamide mixtures or their polymerized products, neat primary or secondary amines and their polymerization products with acrylamide, isopropylacrylamide and N,N methylene bisacrylamide, and glycerine, ethylene glycol, or polyhydroxylic alcohols or mixtures of the above.

The incompatible phase may comprise hydrolyzable phosphate ester or anhydrides and may consist of grafted anhydride or phosphate ester phases blended with or grafted to hydrocarbons such as polypropylenes, polyethylenes, or polystyrenes.

The ionomer composition may be meltable to create a film on a substrate.

The chlorine dioxide is incorporated into n-pentane or other hydrocarbons or low melting chlorowaxes as a vehicle for reaction of the amine containing films to the iminium chlorite containing films.

## Claims

1.   A composition for disinfection, reducing bacterial and fungal growth, and repelling insects comprising:

a first phase containing an iminium chlorite and a hydrogen-bonded matrix; and
a second phase containing a component that is hydrolyzable by atmospheric moisture to an acid, the first and second phases being in intimate contact and incompatible with one another, the first phase being capable of releasing chlorine dioxide upon hydrolysis of the hydrolyzable component on exposure to water.

2.   A composition according to claim 1, wherein the first and second phases are mixed to form a dispersion.

3.   A composition according to claim 1 or claim 2, wherein the iminium chlorite is formed from a primary amine, a secondary amine, or a tertiary amine having hydrogen bonding groups.

4.   A composition according to claim 3, wherein the iminium chlorite is formed from monoethanolamine, diethanolamine, triethanolamine, a linear polymer of 1,3-diaminopropane or 1,2-diaminoethane and N,N-methylene bisacrylamide, 4-dimethylaminopyridine, tetramethylene ethylene diamine, N,N-dimethylamino cyclohexane, 1-(N-dipropylamino)-2-carboxyamido ethane or 1-(N-dimethylamino)-2-carboxyamido ethane.

5.   A composition according to any one of claims 1 to 4, wherein the hydrolyzable component is selected from a carboxylic acid, an ester, an anhydride, an acyl halide, phosphoric acid, a trimethylsilyl phosphate ester, a dialkyl phosphate, a sulfonic acid, a sulfonic acid ester, and a sulfonic acid chloride.

6.   A composition according to claim 5, wherein the hydrolyzable component is an anhydride or phosphate ester blended with or grafted to polypropylene, polyethylene or polystyrene.

7.   A composition according to claim 5 wherein the trimethylsilyl phosphate ester is $(CH_3)_3SiOP(O)(OR)_2$ wherein R is a non-hydrogen bonding group, alkyl or aryl.

8. A composition according to claim 5, wherein the anhydride is a copolymer of maleic anhydride, and styrene or an alkene.

9. A composition according to any one of claims 1 to 8, wherein the second phase contains atactic polypropylene or a low melting polyethylene wax.

10. A composition according to any one of claims 1 to 9, wherein the hydrogen-bonded matrix includes formamide, acrylamide-isopropylacrylamide, glycerine or ethylene glycol.

11. A method of disinfecting, reducing bacterial and fungal growth, repelling insects from and/or deodorizing a substrate comprising:

exposing a substrate to a composition that releases chlorine dioxide in the presence of moisture; and exposing the substrate to moisture to release chlorine dioxide from the composition into the atmosphere surrounding the substrate, the composition being comprised of a first phase containing iminium chlorite and a hydrogen-bonded matrix, and a second phase containing a component that is hydrolyzable by atmospheric moisture to an acid, the first and second phases being in intimate contact and incompatible with one another.

12. A method according to claim 11, wherein the substrate is coated with the composition and the coated substrate is exposed to chlorine dioxide.

13. A method according to claim 12, wherein the substrate is coated with a film comprised of the composition.

14. A process for preparing a composition comprising:

providing a first phase containing an amine and a hydrogen-bonded matrix and a second phase containing a component that is hydrolyzable by atmospheric moisture to an acid, the first and second phases being in intimate contact and incompatible with one another; and exposing the phases to chlorine dioxide that reacts with the amine to form iminium chlorite within the first phase, the first phase being capable of releasing chlorine dioxide upon hydrolysis of the hydrolyzable component.

15. A process according to claim 14, further including the step of applying the first and second phases to a substrate to form a film before exposure to chlorine dioxide.

16. A process according to claim 15, wherein the film is exposed to chlorine dioxide by contacting the film with a gaseous chlorine dioxide atmosphere.

17. A process according to claim 16, wherein the chlorine dioxide is dissolved in an organic solvent that does not absorb water before being exposed to the film.

18. A process according to any one of claims 15 to 17, further including the step of exposing the film to moisture after the film is contacted with chlorine dioxide to hydrolyze the hydrolyzable component and release chlorine dioxide from the film.

19. A process according to any one of claims 14 to 18, wherein the amine is selected from a primary amine, a secondary amine, and a tertiary amine having hydrogen bonding groups.

20. A process according to any one of claims 14 to 19, wherein the hydrolyzable component is selected from a carboxylic acid, an ester, an anhydride, an acyl halide, phosphoric acid, a trimethylsilyl phosphate ester, a dialkyl phosphate, a sulfonic acid, a sulfonic acid ester, and a sulfonic acid chloride.

21. A process according to claim 20, wherein the hydrolyzable component is an anhydride or phosphate ester blended with or grafted to polypropylene, polyethylene or polysytrene.

22. A process according to claim 20, wherein the trimethylsilyl phosphate ester is $(CH_3)_3SiOP(O)(OR)_2$ wherein R is a non-hydrogen bonding group, alkyl or aryl.

**23.** A process according to claim 20, wherein the anhydride is a copolymer of maleic anhydride, and styrene or an alkene.

**24.** A process according to any one of claims 14 to 23, wherein the hydrogen-bonded matrix includes formamide, acrylamideisopropylacrylamide, glycerine or ethylene glycol.

**Patentansprüche**

**1.** Zusammensetzung zur Desinfizierung, Reduzierung des Bakterien- und Pilzwachstums und Abwehr von Insekten mit

einer ersten Phase, die ein Iminiumchlorit und eine wasserstoffgebundene Matrix enthält, und
einer zweiten Phase, die einen Bestandteil enthält, der durch atmosphärische Feuchtigkeit zu einer Säure hydrolysierbar ist, wobei die erste und die zweite Phase untereinander in innigem Kontakt und miteinander unverträglich sind und die erste Phase bei Hydrolyse des hydrolysierbaren Bestandteils infolge Wassereinwirkung zur Freisetzung von Chlordioxid befähigt ist.

**2.** Zusammensetzung nach Anspruch 1, bei der die erste und zweite Phase unter Bildung einer Dispersion gemischt sind.

**3.** Zusammensetzung nach Anspruch 1 oder Anspruch 2, bei der das Iminiumchlorit aus einem primären Amin, einem sekundären Amin oder einem tertiären Amin mit wasserstoffbindenden Gruppen gebildet ist.

**4.** Zusammensetzung nach Anspruch 3, bei der das Iminiumchlorit gebildet ist aus Monoethanolamin, Diethanolamin, Triethanolamin, linearem Polymer von 1,3-Diaminopropan oder 1,2-Diaminoethan und N,N-Methylenbisacrylamid, 4-Dimethylaminopyridin, Tetramethylenethylendiamin, N,N-Dimethylaminocyclohexan, 1-(N-Dipropylamino)-2-carboxyamisoethan oder 1-(N-Dimethylamino)-2-carboxyamidoethan.

**5.** Zusammensetzung nach einem der Ansprüche 1 bis 4, bei der die hydrolysierbare Komponente unter einer Carbonsäure, einem Ester, einem Anhydrid, einem Acylhalogenid, Phosphorsäure, einem Trimethylsilylphosphatester, einem Dialkylphosphat, einer Sulfonsäure, einem Sulfonsäureester und einem Sulfonsäurechlorid ausgewählt ist.

**6.** Zusammensetzung nach Anspruch 5, bei der die hydrolysierbare Komponente ein Anhydrid oder Phosphatester ist, die mit Polypropylen, Polyethylen oder Polystyrol gemischt oder gepfropft sind.

**7.** Zusammensetzung nach Anspruch 5, bei der der Trimethylsilylphosphatester $(CH_3)_3SiOP(O)(OR)_2$ ist, worin das R eine nichtwasserstoffbindende Gruppe, Alkyl oder Aryl ist.

**8.** Zusammensetzung nach Anspruch 5, bei der das Anhydrid ein Copolymer von Maleinsäureanhydrid und Styrol oder einem Alken ist.

**9.** Zusammensetzung nach einem der Ansprüche 1 bis 8, bei der die zweite Phase ataktisches Polypropylen oder ein niedrigschmelzendes Polyethylenwachs enthält.

**10.** Zusammensetzung nach einem der Ansprüche 1 bis 9, bei der die wasserstoffgebundene Matrix Formamid, Acrylamid-Isopropylacrylamid, Glycerin oder Ethylenglykol enthält.

**11.** Verfahren zum Desinfizieren, Reduzieren des Bakterien- und Pilzwachstums, Abwehren von Insekten und/oder Desodorieren eines Substrats, bei dem man

ein Substrat einer Zusammensetzung aussetzt, die in Gegenwart von Feuchtigkeit Chlordioxid freisetzt, und
das Substrat der Feuchtigkeit aussetzt, um aus der Zusammensetzung Chlordioxid in die das Substrat umgebende Atmosphäre freizusetzen, wobei die Zusammensetzung aus einer Iminiumchlorit und eine wasserstoffgebundene Matrix enthaltenden ersten Phase und einer einen durch atmosphärische Feuchtigkeit zu einer Säure hydrolysierbaren Bestandteil enthaltenden zweiten Phase besteht, wobei die erste und zweite Phase in innigem Kontakt und unverträglich miteinander sind.

**12.** Verfahren nach Anspruch 11, bei dem das Substrat mit der Zusammensetzung beschichtet und das beschichtete Substrat dem Chlordioxid ausgesetzt wird.

**13.** Verfahren nach Anspruch 12, bei dem man das Substrat mit einem die Zusammensetzung enthaltenden Film beschichtet.

**14.** Verfahren zur Herstellung einer Zusammensetzung, bei dem man eine ein Amin und eine wasserstoffgebundene Matrix enthaltende erste Phase und eine eine durch atmosphärische Feuchtigkeit zu einer Säure hydrolysierbare Komponente enthaltende zweite Phase vorsieht, wobei die erste und zweite Phase in innigem Kontakt und miteinander unverträglich sind, und die Phasen dem Chlordioxid aussetzt, das mit dem Amin unter Bildung von Iminiumchlorit in der ersten Phase reagiert, wobei die erste Phase bei Hydrolyse des hydrolysierbaren Bestandteils zur Freisetzung von Chlordioxid befähigt ist.

**15.** Verfahren nach Anspruch 14, ferner mit der Stufe der Aufbringung der ersten und zweiten Phase auf ein Substrat, um vor der Chlordioxid-Einwirkung einen Film zu bilden.

**16.** Verfahren nach Anspruch 15, bei dem der Film dem Chlordioxid dadurch ausgesetzt wird, daß der Film mit einer gasförmigen Chlordioxidatmosphäre in Kontakt gebracht wird.

**17.** Verfahren nach Anspruch 16, bei dem das Chlordioxid in einem organischen Lösungsmittel gelöst wird, das kein Wasser absorbiert, bevor es dem Film ausgesetzt wird.

**18.** Verfahren nach einem der Ansprüche 15 bis 17, ferner mit der Stufe, in der der Film der Feuchtigkeit ausgesetzt wird, nachdem er mit Chlordioxid in Kontakt gebracht wurde, um den hydrolysierbaren Bestandteil zu hydrolysieren und Chlordioxid aus dem Film freizusetzen.

**19.** Verfahren nach einem der Ansprüche 14 bis 18, bei dem das Amin unter einem primären Amin, einem sekundären Amin und einem tertiären amin mit wasserstoffbindenden Gruppen ausgewählt wird.

**20.** Verfahren nach einem der Ansprüche 14 bis 19, bei dem der hydrolysierbare Bestandteil unter einer Carbonsäure, einem Ester, einem Anhydrid, einem Acylhalogenid, Phosphorsäure, einem Trimethylsilylphosphatester, einem Dialkylphosphat, einer Sulfonsäure, einem Sulfonsäureester und einem Sulfonsäurechlorid ausgewählt wird.

**21.** Verfahren nach Anspruch 20, bei dem der hydrolgsierbare Bestandteil ein Anhydrid oder Phosphatester ist, der mit Polypropylen, Polyethylen oder Polystyrol gemischt oder gepfropft ist.

**22.** Verfahren nach Anspruch 20, bei dem der Trimethylsilylphosphatester $(CH_3)_3SiOP(O)(OR)_3$ ist, worin R eine nichtwasserstoffbindende Gruppe, Alkgl oder Aryl ist.

**23.** Verfahren nach Anspruch 20, bei dem das Anhydrid ein Copolymer aus Maleinsäureanhydrid und Styrol oder einem Alken ist.

**24.** Verfahren nach einem der Ansprüche 14 bis 23, bei dem die wasserstoffgebundene Matrix Formamid, Acrylamid-Isopropglacrylamid, Glycerin oder Ethylenglycol enthält.

## Revendications

**1.** Composition pour désinfecter, réduire la croissance bactérienne et fongique et éloigner les insectes comprenant :

une première phase contenant un chlorite d'iminium et une matrice à liaison hydrogène; et
une seconde phase contenant un constituant qui peut être hydrolysé en un acide par l'humidité atmosphérique, la première et la seconde phase étant en contact intime et incompatibles l'une avec l'autre, la première phase étant capable de libérer du dioxyde de chlore, par hydrolyse du constituant que l'on peut hydrolyser par exposition à de l'eau.

**2.** Composition selon la revendication 1, dans laquelle la première et la seconde phase sont mélangées afin de former une dispersion.

**3.** Composition selon la revendication 1 ou la revendication 2, dans laquelle le chlorite d'iminium est formé à partir d'une amine primaire, d'une amine secondaire ou d'une amine tertiaire comportant des groupes à liaison hydrogène.

4. Composition selon la revendication 3, dans laquelle le chlorite d'iminium est formé à partir de là monoéthanolamine, de la diéthanolamine, de la triéthanolamine, d'un polymère linéaire de 1,3-diaminopropane ou de 1,2-diaminoéthane et de N,N-méthylènebisacrylamide, de la 4-diméthylaminopyridine, de la tétraméthylèneéthylènediamine, du N,N-diméthylaminocyclohexane, du 1-(N-dipropylamino)-2-carboxyamidoéthane ou du 1-(N-diméthylamino)-2-carboxyamidoéthane.

5. Composition selon l'une quelconque des revendications 1 à 4, dans laquelle le constituant que l'on peut hydrolyser est choisi parmi un acide carboxylique, un ester, un anhydride, un halogénure d'acyle, un acide phosphorique, un triméthylsilylphosphate, un phosphate de dialkyle, un acide sulfonique, un ester d'acide sulfonique et un chlorure d'acide sulfonique.

6. Composition selon la revendication 5, dans laquelle le constituant que l'on peut hydrolyser est un anhydride ou un phosphate mélangé avec ou greffé sur un polypropylène, un polyéthylène ou un polystyrène.

7. Composition selon la revendication 5, dans laquelle le triméthylsilylphosphate est $(CH_3)_3SiOP(O)(OR)_2$ dans lequel R est un coupe qui n'est pas à liaison hydrogène, un groupe alkyle ou aryle.

8. Composition selon la revendication 5, dans laquelle l'anhydride est un copolymère d'anhydride maléique et de styrène ou d'un alcène.

9. Composition selon l'une quelconque des revendications 1 à 8, dans laquelle la seconde phase contient un polypropylène atactique ou une cire de polyéthylène à bas point de fusion.

10. Composition selon l'une quelconque des revendications 1 à 9, dans laquelle la matrice à liaison hydrogène comprend le formamide, un mélange acrylamide - isopropylacrylamide, le glycérol ou l'éthylèneglycol.

11. Procédé pour désinfecter un substrat, réduire la croissance bactérienne et fongique sur un substrat, éloigner les insectes d'un substrat et/ou désodoriser un substrat comprenant les étapes suivantes :

   exposition d'un substrat à une composition qui libère du dioxyde de chlore en présence d'humidité; et exposition du substrat à l'humidité afin de libérer, dans l'atmosphère entourant le substrat, du dioxyde de chlore à partir de la composition, la composition étant constituée d'une première phase contenant un chlorite d'iminium et une matrice à liaison hydrogène et d'une seconde phase contenant un constituant qui peut être hydrolysé en un acide par l'humidité atmosphérique, la première phase et la seconde phase étant en contact intime et incompatibles l'une avec l'autre.

12. Procédé selon la revendication 11, dans lequel le substrat est revêtu avec la composition et le substrat revêtu est exposé à du dioxyde de chlore.

13. Procédé selon la revendication 12, dans lequel le substrat est revêtu avec un film constitué de la composition.

14. Procédé pour préparer une composition comprenant les étapes suivantes :

   obtention d'une première phase contenant une amine et une matrice à liaison hydrogène et d'une seconde phase contenant un constituant qui peut être hydrolysé en un acide par l'humidité atmosphérique, la première phase et la seconde phase étant en contact intime et incompatibles l'une avec l'autre; et exposition des phases à du dioxyde de chlore qui réagit avec l'amine pour former du chlorite d'iminium à l'intérieur de la première phase, la première phase étant capable de libérer du dioxyde de chlore par hydrolyse du constituant que l'on peut hydrolyser.

15. Procédé selon la revendication 14, comprenant en outre l'étape consistant à appliquer la première et la seconde phase sur un substrat afin de former un film avant l'exposition au dioxyde de chlore.

16. Procédé selon la revendication 15, dans lequel le film est exposé à du dioxyde de chlore par mise en contact du film avec une atmosphère constituée par du dioxyde de chlore gazeux.

**17.** Procédé selon la revendication 16, dans lequel le dioxyde de chlore est dissous dans un solvant organique qui n'absorbe pas l'eau avant d'être exposé au film.

**18.** Procédé selon l'une quelconque des revendications 15 à 17, comprenant en outre l'étape consistant à exposer le film à l'humidité après avoir mis en contact le film avec du dioxyde de chlore afin d'hydrolyser le constituant que l'on peut hydrolyser et de libérer le dioxyde de chlore à partir du film.

**19.** Procédé selon l'une quelconque des revendications 14 à 18, dans lequel l'amine est choisie parmi une amine primaire, une amine secondaire et une amine tertiaire comportant des groupes à liaison hydrogène.

**20.** Procédé selon l'une quelconque des revendications 14 à 19, dans lequel le constituant que l'on peut hydrolyser est choisi parmi un acide carboxylique, un ester, un anhydride, un halogénure d'acyle, un acide phosphorique, un triméthylsilylphosphate, un phosphate de dialkyle, un acide sulfonique, un ester d'acide sulfonique et un chlorure d'acide sulfonique.

**21.** Procédé selon la revendication 20, dans lequel le constituant que l'on peut hydrolyser est un anhydride ou un phosphate mélangé avec ou greffé sur un polypropylène, un polyéthylène ou un polystyrène.

**22.** Procédé selon la revendication 20, dans lequel le triméthylsilylphosphate est $(CH_3)_3SiOP(O)(OR)_2$ dans lequel R est un groupe qui n'est pas à liaison hydrogène, un groupe alkyle ou aryle.

**23.** Procédé selon la revendication 20, dans lequel l'anhydride est un copolymère d'anhydride maléique et de styrène ou d'un alcène.

**24.** Procédé selon l'une quelconque des revendications 14 à 23, dans lequel la matrice à liaison hydrogène comprend le formamide, un mélange acrylamide - isopropylacrylamide, le glycérol ou l'éthylèneglycol.

Figure 1a. Preparation of Iminium Chlorite Film.

13

Figure 1b.  Release of Chlorine Dioxide from Iminium Chlorite
Film Upon Exposure to Water.